# EUROPEAN PATENT APPLICATION

(11) **EP 2 740 442 A2**
(43) Date of publication of application: **11.06.2014**
(21) Application number: 12817505.6
(22) Date of filing: 29.06.2012
(51) Int. Cl.: A61F 2/10, A61B 17/00

(54) **HAIR TRANSPLANT DEVICE**

(30) Priority: 28.07.2011 KR 20110074856; 18.06.2012 KR 20120064865
(71) Applicant: Oc, Kun, Seoul 137-040 (KR); Kim, Kwang Soo, Seoul 122-081 (KR)
(72) Inventor: Oc, Kun, Seoul 137-040 (KR); Kim, Kwang Soo, Seoul 122-081 (KR)
(74) Representative: Dr. Weitzel & Partner
(86) International application number: PCT/KR2012/005191
(87) International publication number: WO 2013/015534

(57) **Abstract**

Disclosed is a hair transplanter which may finely adjust and set the length of a hair transplantation tool, into which a hair root is inserted, according to thicknesses of skin tissue layers within the scalps of individuals during hair transplantation and thus be conveniently used.

## Description

### BACKGROUND OF THE INVENTION

### Field of the Invention

The present invention relates to a hair transplanter to transplant hair to a balding part, and more particularly to a hair transplanter which may finely adjust the length of a hair transplantation tool, into which a hair root is inserted, according to thicknesses of skin tissue layers within the scalps of individuals during hair transplantation.

### Description of the Related Art

A hair transplanter is an apparatus used to transplant natural hair or artificial hair to a balding part of the skin of a human body where hair is lost due to diseases or accidents. In general, the hair transplanter is used to transplant eyelashes, eyebrows, beard hair, pubic hair and hair of the head. In case of a single hair transplantation method which has been conventionally used, hair roots are separated from a human body one by one and transplanted into a desired region using a needle of a hair transplantation tool, thus preventing bleeding and scars. Thereby, direction, angle and density of hair may be arbitrarily adjusted and thus the shape of hair after surgery is naturally maintained. Therefore, the conventional single hair transplantation method has been widely used in transplantation of eyelashes, eyebrows, beard hair and pubic hair as well as hair of the head. In case of conventional hair transplanters, when a hair root connected to hair is located on the inner front surface of a hair transplantation tool of the hair transplanter and then the hair transplantation tool is inserted into the scalp, the hair transplantation tool forms a hole and the hair root is received within the scalp along the hair transplantation tool. At this time, in order to push the hair root into skin tissue within the scalp, a shaft moving so as to be slidable within the hair transplantation tool may be used, or a guide plate serving as the shaft and protruding from the front surface of a sliding member may be used so that the hair transplantation tool is retreated backward and is not moved forward any more by the guide plate.

In most conventional hair transplanters, the length of a hair transplantation tool is fixed when the hair transplanter is initially manufactured. If hair roots are transplanted into the scalp, since skin tissues within the human scalp have different thicknesses, an operator performs hair transplantation while arbitrarily adjusting the length of the hair transplantation tool inserted into the scalp depending on experience and may thus cause damage to the skin tissues within the scalp and generate excessive bleeding or inflammation. Recently, a hair transplanter in which the length of a hair transplantation tool may be adjusted has been developed, but is not widely used now due to the complicated structure and high price thereof.

In conventional hair transplanters, since scalp layers of individuals differ according to their scalps, it is difficult for an operator to finely adjust the length of a hair transplantation tool of the hair transplanter according to the positions of the tops of the heads of individuals while performing transplantation, and the operator needs to adjust the length of the hair transplantation tool one by one and thus a long setting time is taken.

### SUMMARY OF THE INVENTION

Therefore, the present invention has been made in view of the above problems, and it is an object of the present invention to provide a hair transplanter which may conveniently adjust the length of a hair transplantation tool, into which a hair root is inserted, according to thicknesses of skin tissue layers within the scalps of individuals during hair transplantation so as to prevent damage to skin tissues in the scalp, to stably receive the hair root within the scalp, and to transplant the hair root to a desired depth within the scalp to shorten a time taken to perform hair transplantation.

It is another object of the present invention to provide a hair transplanter having high precision which may finely adjust the lengths of a hair transplantation tool and a shaft and have a locking function of the hair transplantation tool and the shaft, as needed, during hair transplanation.

In accordance with an aspect of the present invention, the above and other objects can be accomplished by the provision of a hair transplanter including a central rod provided with an upper protrusion protruding from the upper end thereof and a lower protrusion of a conical shape formed at the lower end thereof, a central case body provided with an angle cover of a conical shape formed at the lower end thereof, a tuning cover combined with the upper surface of the central case body and adjusting the length of the central case body in the longitudinal direction, and a fixing cover mounted to fix the tuning cover, the central rod, into which a hair transplantation tool and a shaft are sequentially inserted, being mounted within the central case body, and a pressing button unit combined with the upper protrusion of the central rod, on which a spring member inserted into the central case body so as to be movable forward by external force and combined with the shaft so as to be restored to an original position thereof by force applied from the outside is formed, wherein the pressing button unit includes a fine adjustment lever provided with a space in which a head of the shaft is received so as to finely adjust the length of the shaft inserted into the hair transplantation tool and a lock nut fixed in front of the fine adjustment lever in a finely adjusted state.

### BRIEF DESCRIPTION OF THE DRAWINGS

The above and other objects, features and other advantages of the present invention will be more clearly understood from the following detailed description taken in conjunction with the accompanying drawings, in which:
FIG. 1 is a perspective view of a hair transplanter having high precision in accordance with one embodiment of the present invention;
FIG. 2 is an exploded perspective view of the hair transplanter having high precision in accordance with the embodiment of the present invention;
FIG. 3 is a cross-sectional view of the hair transplanter having high precision in accordance with the embodiment of the present invention;
FIG. 4 is a cross-sectional view illustrating an operating state of the hair transplanter having high precision in accordance with the embodiment of the present invention; and
FIG. 5 is a cross-sectional view of a hair transplanter having high precision in accordance with another embodiment of the present invention.

### DETAILED DESCRIPTION OF THE INVENTION

Now, preferred embodiments in accordance with the present invention will be described in detail with reference to the annexed drawings.

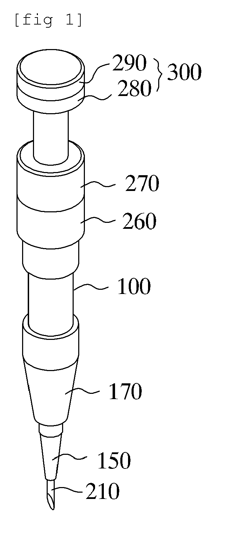

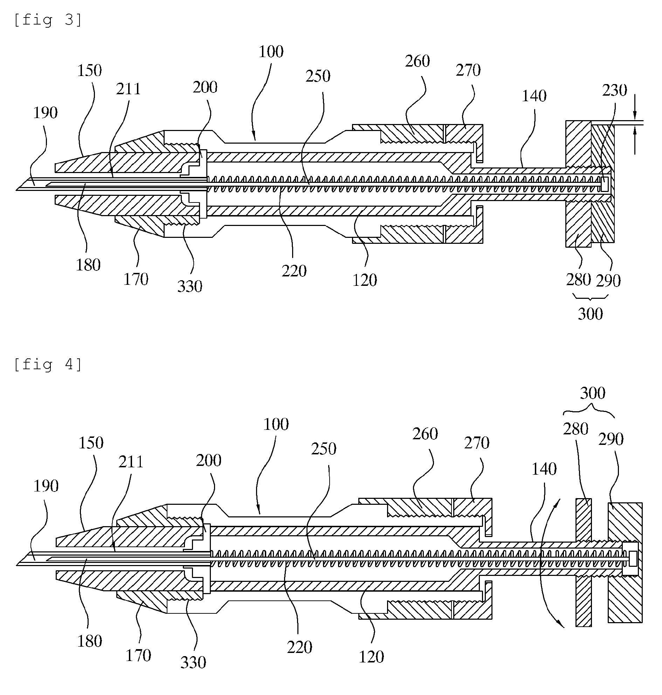

As exemplarily shown in FIGs. 1 to 5, a hair transplanter having high precision in accordance with one embodiment of the present invention is configured such that a hair transplantation tool 210 includes a tubular needle 180 provided with an insertion hole 190 formed therein and a hair fixing groove formed on the side surface thereof, and a through hole formed at the upper end of the needle 180 and tapered in the inward direction so that the lower end of a shaft 250 may be easily inserted into the through hole and thus fixed.

The hair transplanter having high precision in accordance with the embodiment of the present invention includes a central rod 120 provided with an upper protrusion protruding from the upper end thereof and a lower protrusion 150 of a conical shape formed at the lower end thereof, a central case body 100 provided with an angle cover 170 of a conical shape formed at the lower end thereof, a tuning cover 270 combined with the upper surface of the central case body 100 and adjusting the length of the central case body 100 in the longitudinal direction, and a fixing cover 260 mounted to fix the tuning cover 270, the central rod 120, into which a hair transplantation tool 210 and a shaft 250 are sequentially inserted, being mounted within the central case body 100, and a pressing button unit 300 combined with the upper protrusion 140 of the central rod 120, on which a spring member 220 inserted into the central case body 100 so as to be movable forward by external force and combined with the shaft 250 so as to be restored to an original position thereof by force applied from the outside is formed, the pressing button unit 300 includes a fine adjustment lever 290 provided with a space in which a head 240 of the shaft 250 is received so as to finely adjust the length of the shaft 250 inserted into the hair transplantation tool 210 and a lock nut 280 fixed in front of the fine adjustment lever 290 in a finely adjusted state.

The central rod 120 forms a cylindrical extension protruding to a designated length so as to be inserted into a hollow 110 of the central case body 100, and has the same width as the width of a guide hole 160 so as to be movable in the forward and backward longitudinal directions within a space of the guide hole 160.

The hair transplantation tool 210, the shaft 250, the spring member 220, and the central rod 120 are sequentially inserted into the central case body 100. In the hair transplanter in accordance with the embodiment of the present invention, the angle cover 170 of a conical shape is combined with a combination groove of the central case body 100. A circular combination protrusion protrudes from the outer circumferential surface of the lower portion of the angle cover 170 and is combined with the inside of the central case body 100 and thus, the angle cover 170 may be effectively separated from the central case body 100.

In the hair transplanter in accordance with the embodiment of the present invention, since the angle cover 170 is manufactured separately from the central case body 100 and combined with the central case body 100, the central rod 120 inserted into the central case body 100 and the hair transplantation tool 210 inserted into the central rod 120 may be simply replaced.

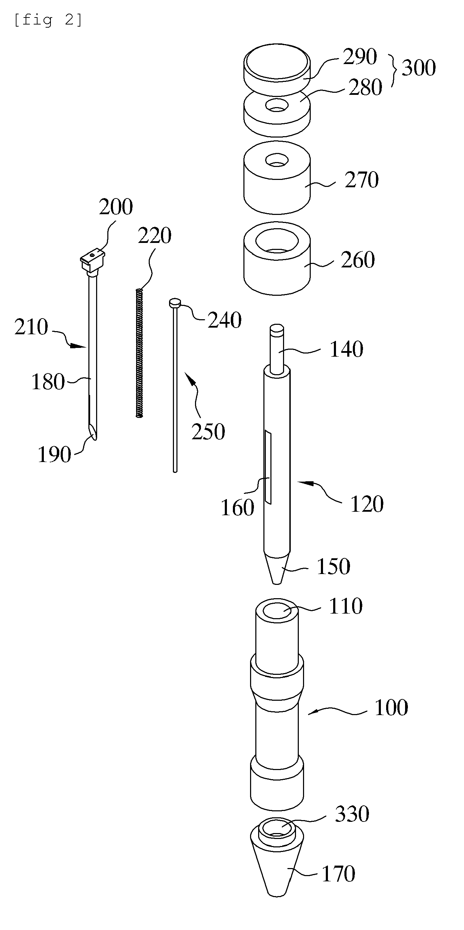

The above-described hair transplanter in accordance with the embodiment of the present invention is assembled, as exemplarily shown in FIG. 2.

The hair transplanter includes the central case body 100 provided with the hollow 110 passing through both ends thereof and formed in the longitudinal direction so that the central rod 120, with which the spring member 220, the shaft 250, and the hair transplantation tool 210 are combined, is mounted in the hollow 110, the central rod 120 provided with the upper protrusion 140 of a cylindrical shape protruding from the upper end thereof and the lower protrusion 150 of a conical shape formed at the lower end thereof, the angle cover 170 of a conical shape combined with the inside of the lower end of the central case body 100 and provided with an insertion hole 330 formed at the center of the inside thereof in the longitudinal direction, the hair transplantation tool 210 including the needle 180 combined with a needle guide 200, the lower end of the hair transplantation tool 210 being sharpened and inserted into the conical angle cover 170 so as to protrude outward, the tuning cover 270 combined with the upper portion of the central case body 100 so as to adjust the length of the central case body 100 in the longitudinal direction, the fixing cover 260 mounted in front of the tuning cover 270 so as to fix the tuning cover 270, the shaft 250 inserted into the central rod 120, inserted into the upper end of the hair transplantation tool 210, and projecting and retracting by a designated length, the spring member 220 inserted into the central case body 100 so as to be movable forward by external force and interposed between the central rod 120 and the shaft 250 so as to restore the hair transplantation tool 210, moved forward by force applied from the outside, to an original position thereof, and the pressing button unit 300 coupled with the upper protrusion 140 of the central rod 120.

The fixing cover 260 and the tuning cover 270 are configured so as to adjust the length of the pressing button unit 300, and the fine adjustment lever 290 in which the head 240 of the shaft 250 is received and the lock nut 280 formed on the fine adjustment lever 290 are provided so as to finely adjust the length of the shaft 250 inserted into the hair transplantation tool 210.

Further, the lock nut 280 has a larger size (diameter) than the fine adjustment lever 290 so as to be fixed on the upper portion of the fine adjustment lever 290 in a finely adjusted state. Since, if the lock nut 280 and the fine adjustment lever 290 have the same size, it may be difficult to turn the lock nut 280 separately from the fine adjustment lever 290, and the lock nut 280 may have a larger diameter than the fine adjustment lever 290.

The thicknesses of the scalps of individuals are different, and the thicknesses of the scalp of an individual are different according to positions of the head of the individual. Since such a thickness difference is not great, fine adjustment is required. Therefore, in order to finely adjust the length of the shaft 250, fine adjustment of the pressing button unit 300 is carried out and the lock nut 280 and the fine adjustment lever 290 are formed to fix the pressing button unit 300 after fine adjustment.

An operator moves the shaft 250 to a part to be operated during operation by rotating the fine adjustment lever 290 and, in order to set such a position of the shaft 250, fixes the fine adjustment lever 290 by rotating the lock nut 280.

Further, in order to change the position of the shaft 250, the operator loosens the lock nut 280, moves the fine adjustment lever 290 to a desired position, and then fixes the fine adjustment lever 290 by tightening the lock nut 280.

Through such fine adjustment, the operator may conveniently adjust the length and position of the shaft 250 and then fix the adjusted position of the shaft 250.

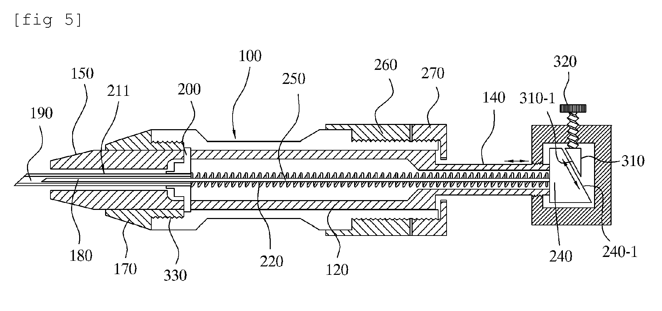

In a hair transplanter having high precision in accordance with another embodiment of the present invention, as exemplarily shown in FIG. 5, in order to adjust the length of hair fixed to the scalp during hair transplantation, a tuning cover 270 is adjusted and then fixed using a fixing cover 260 so that the moving distance of a lower protrusion 150 may be adjusted. Since an operator needs to perform fine adjustment with respect to the scalp according to the part of the head and fix the operating distance of a shaft 250 set during operation, a through hole is formed on the side surface of a pressing button unit 300 and a screw thread is formed on the inner surface of the through hole, a wedge adjustment lever 320 including a wedge 310 provided with an inclined surface 310-1 formed at the end thereof is formed, and a head 240 of the shaft 250 provided with an inclined surface moving in the upward and downward directions along the wedge 310 provided with the inclined surface 310-1 is formed.

In the hair transplanter in accordance with this embodiment of the present invention, the operating length of the shaft 250 may be finely adjusted by moving the shaft 250 along the inclined surface 130-1 of the wedge 310 connected to the wedge adjustment lever 320 and the inclined surface 240-1 of the head 240 of the shaft 250 through butt joint.

When the wedge adjustment lever 320 is rotated in the clockwise direction, the wedge 310 moves forward and the head 240 provided with the inclined surface 240-1 moves forward by the wedge 310 and, when the wedge adjustment lever 320 is rotated in the counterclockwise direction, the wedge 310 moves backward. Thereby, fine adjustment may be achieved.

In accordance with this embodiment of the present invention, scale marks are formed on the hair transplantation tool 210 and the shaft 250 and thus, an operator may visually confirm whether or not hair is uniformly inserted into a skin tissue layer within the scalp of an individual.

As apparent from the above description, one embodiment of the present invention provides a hair transplanter which may finely adjust the length of a hair transplantation tool, into which a hair root is inserted, according to thicknesses of a skin tissue layer within the scalp of an individual during hair transplantation, have a locking function of the hair transplantation tool, and thus be conveniently operated.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. A hair transplanter having high precision comprising:
a central rod (120) provided with an upper protrusion protruding from the upper end thereof and a lower protrusion (150) of a conical shape formed at the lower end thereof;
a central case body (100) provided with an angle cover (170) of a conical shape formed at the lower end thereof, a tuning cover (270) combined with the upper surface of the central case body (100) and adjusting the length of the central case body (100) in the longitudinal direction, and a fixing cover (260) mounted to fix the tuning cover (270), the central rod (120), into which a hair transplantation tool (210) and a shaft (250) are sequentially inserted, being mounted within the central case body (100); and
a pressing button unit (300) combined with the upper protrusion (140) of the central rod (120), on which a spring member (220) inserted into the central case body (100) so as to be movable forward by external force and combined with the shaft (250) so as to be restored to an original position thereof by force applied from the outside is formed,
wherein the pressing button unit (300) includes a fine adjustment lever (290) provided with a space in which a head (240) of the shaft (250) is received so as to finely adjust the length of the shaft (250) inserted into the hair transplantation tool (210) and a lock nut (280) fixed in front of the fine adjustment lever (290) in a finely adjusted state.

2. The hair transplanter having high precision according to claim 1, wherein:
the pressing button unit (300) further includes a wedge adjustment lever (320) mounted on the side surface of the pressing button unit (300) and a wedge (310) provided with an inclined surface (310-1) and connected to the wedge adjustment lever (320); and
the head (240) of the shaft (250) is provided with an inclined surface (240-1), so that the operating length of the shaft (250) may be finely adjusted by moving the head (240) of the shaft (250) along the inclined surface (130-1) and the inclined surface (240-1) adhered to each other through butt joint.

3. The hair transplanter having high precision according to claim 1, wherein the diameter of the lock nut (280) is greater or smaller than the fine adjustment lever (290) so as to be fixed to the upper surface of the fine adjustment lever (290) in a finely adjusted state.

4. The hair transplanter having high precision according to claim 1, wherein scale marks are formed on the hair transplantation tool (210) and the shaft (250).
